# FASCICULE DE BREVET EUROPEEN

(11) **EP 0 637 317 B1**
(45) Date de publication et mention de la délivrance du brevet: **13.08.2003**
(21) Numéro de dépôt: 93911814.7
(22) Date de dépôt: 22.04.1993
(51) Int. Cl.: C07K 14/76, C07K 1/16

(54) **PROCEDE DE PURIFICATION D'UNE SOLUTION AQUEUSE D'ALBUMINE BRUTE**
VERFAHREN ZUR REINIGUNG EINER ROHEN ALBUMINWÄSSERIGEN LÖSUNG
METHOD FOR PURIFYING AN AQUEOUS SOLUTION OF RAW ALBUMIN

(30) Priorité: 22.04.1992 FR 9204941
(43) Date de publication de la demande: 08.02.1995
(73) Titulaire: RUCHETON, Marcel, F-34000 Montpellier (FR); STEFAS, Elie, F-34280 La Grande-Motte (FR); GRAAFLAND, Hubert, F-34000 Montpellier (FR)
(72) Inventeur: RUCHETON, Marcel, F-34000 Montpellier (FR); STEFAS, Elie, F-34280 La Grande-Motte (FR); GRAAFLAND, Hubert, F-34000 Montpellier (FR)
(74) Mandataire: Peuscet, Jacques
(86) Numéro de dépôt international: FR9300395
(87) Numéro de publication internationale: WO93021228

(56) Documents cités:
- EP-A- 0 319 067
- EP-A- 0 366 946
- BIOCHIMICA ET BIOPHYSICA ACTA vol. 372, no. 1, 1974, AMSTERDAM pages 218 - 224 WICHMAN A. ET AL 'Purification of human serum albumin by affinity chromatography'
- SCOPES R.K. 'Protein purification. Principles and practice' 1987 , SPRINGER VERLAG , NEW YORK
- GENERAL PHYSIOLOGY AND BIOPHYSICS vol. 5, no. 2, 1986, BRATISLAVA pages 201 - 204 KRIZANOVA, O. ET AL 'Application of a new hydrophobic carrier for routine isolation of calmodulin and other proteins'
- 'Dictionnaire de la chimie et de ses application', DUVAL, 3° EDITION TECHNIQUE ET DOCUMENTATION * page 425 *

## Description

L'invention concerne un procédé de purification d'une solution aqueuse d'albumine brute.

La solution peut être toute solution aqueuse d'albumine brute. Elle est plus particulièrement une solution d'albumine obtenue à partir du plasma sanguin d'un mammifère tel que bovin, cheval, porc, mouton ou lapin, et, de préférence, un solution obtenue à partir de plasma humain.

Il est bien connu de séparer à partir de sang, par centrifugation, d'une part, les globules et, d'autre part, le plasma sanguin. On sait depuis de nombreuses années que le plasma contient, outre l'albumine qui constitue la majeure partie des matières dissoutes, toute une série d'autres molécules, notamment des protéines, dont l'extraction est particulièrement intéressante compte tenu de leurs utilisations thérapeutiques. On a donc proposé des procédés de précipitation fractionnée par un agent de précipitation à des pH, forces ioniques et température variables. La méthode la plus largement utilisée à l'heure actuelle est celle de Cohn (voir COHN J. et autres J. Am. Chem. Soc. 72,465-474/1950) ou des variantes de cette méthode où l'agent de précipitation utilisé est l'éthanol. Selon ladite méthode de Cohn, le plasma est refroidi à - 30° C puis réchauffé à + 2° C, d'où il en résulte un cryoprécipité contenant le facteur VIII anti-hémophilique, le fibrinogène et la fibronectine. Le surnageant, dit "surnageant I", est séparé du précipité susmentionné ; on abaisse son pH à 5,85 ± 0,05 et on ajoute de l'éthanol jusqu'à ce que la concentration éthanolique soit de 19 % en volume, la température étant abaissée, au fur et à mesure de l'addition d'éthanol, jusqu'à - 5° C. On obtient ainsi un précipité, dit "précipité (II + III)", contenant notamment les gamma-globulines et un surnageant, dit "surnageant (II + III)" contenant l'albumine et des impuretés. On reprend le surnageant ainsi obtenu et on augmente le taux d'alcool jusqu'à obtenir une concentration éthanolique de 40 % en volume, la température étant abaissée à environ - 8° C. On obtient ainsi un précipité, dit "précipité IV", et un surnageant, dit "surnageant IV", qui contient l'albumine à un degré de pureté d'environ 94 à 97 % en poids de protéines. Le surnageant IV sert de matière première pour les solutions d'albumine désirées, mais il contient une grande quantité d'éthanol ; selon une première technique on peut dialyser directement le surnageant IV contre du sérum physiologique, mais il faut alors utiliser une très grande quantité d'eau et le procédé est donc lent et coûteux ; selon une autre technique, on abaisse le pH du surnageant IV à 4,80 ± 0,05 ce qui fait précipiter l'albumine : on sépare ce précipité, dit "précipité V", et on le remet en solution dans du sérum physiologique, le reste de l'éthanol étant extrait par dialyse.

Pour compléter la purification de l'albumine, on a déjà proposé d'utiliser des techniques chromatographiques, notamment par échange d'ions (voir notamment Curling J.M., Methods of Plasma Protein Fractionation, Ed. J. Curling, Acad. Press, 77-91 (1980) ; Tayot J.L. et autres, Methods of Plasma Protein Fractionation, Ed. J. Curling, Acad. Press, 149-160 (1980) ; Saint-Blancard J., Nouveaux échangeurs d'ions Trisacryl, Ann. Pharm. Fr. 39, 403-409 (1981)). Le principe de ces méthodes est de fixer l'albumine sur un support spécifique, en fonction du pH et de la force ionique, et d'éliminer ainsi dans l'effluent certaines des impuretés qui se trouvent dans la solution d'albumine traitée par chromatographie. Malheureusement, si ces techniques permettent d'améliorer de façon sensible la qualité des solutions d'albumine obtenues, par contre, elles sont difficiles à mettre en oeuvre et onéreuses. En effet, les solutions d'albumine traitées contiennent approximativement 4 % d'impuretés diverses à extraire. Etant donné que l'on fixe l'albumine sur le remplissage de la colonne de chromatographie, il faut fixer 96 % de la matière traitée et comme la fixation d'albumine par gramme de remplissage est limitée, de l'ordre de grandeur de 10 mg environ, cela implique la mise en oeuvre d'un très gros volume de lit de remplissage dans les colonnes. Etant donné que le remplissage est très volumineux, la mise en oeuvre exige une grande quantité d'eau pour assurer d'une part, le prééquilibrage de la colonne et d'autre part, le passage du produit sur la colonne ; en outre, la nature des remplissages utilisés exigent généralement, pour le prééquilibrage, l'utilisation de tampons spéciaux. Par ailleurs, lorsque l'albumine a été retenue dans le lit de remplissage, il faut, pour la récupérer, procéder à son élution de la colonne au moyen de solutions-tampons appropriées, de sorte que l'albumine se retrouve ensuite mélangée à ces tampons et qu'il convient de redialyser les solutions d'albumine pour faire disparaître les sels des solutions-tampons et obtenir finalement un produit injectable.

Il est également connu, par exemple par EP-A 0 367 220, de séparer les impuretés par passage sur une colonne de DEAE-SEPHADEX ou sur une colonne de SEPHAROSE 4B activée par du bromure de cyanogène sur lequel sont fixés des anticorps spécifiques, les impuretés telles que l'haptoglobine et α₁-AGP se fixant sur la colonne et l'effluent étant constitué par l'albumine purifiée. Ce type de chromatographie est avantageux car, en premier lieu, seules les impuretés doivent être retenues sur la colonne de chromatographie, ce qui permet d'utiliser des lits de remplissage de volume réduit ; en deuxième lieu, le volume d'effluent contenant l'albumine est également réduit, en effet, il est de l'ordre du volume de charge initiale ; en troisième lieu, le prééquilibrage des colonnes ne nécessite ainsi que des quantités de liquide réduites ; en quatrième lieu, il n'y a pas à laver les lits de remplissage de façon précautionneuse et complète pour la récupération de l'albumine et en cinquième lieu il suffit de régénérer les colonnes de chromatographie par simple lavage, sans besoin de grandes précautions pour les méthodes de lavage. Mais l'albumine obtenue peut encore former à la chaleur des quantités trop importantes de polymères.

La présente invention a pour objet un procédé de purification dans lequel on fixe les impuretés par chromatographie, l'effluent étant constitué par une solution d'albumine qui permet d'obtenir une albumine très pure, ne formant pratiquement pas de polymères à la chaleur.

La présente invention concerne un procédé de purification d'une solution aqueuse d'albumine brute dans lequel on fixe des protéines contaminantes par chromatographie sur une phase stationnaire solide, la solution d'albumine purifiée étant recueillie comme effluent, caractérisé par le fait qu'on choisit comme phase stationnaire, une phase neutre ou voisine de la neutralité chargée d'au moins un composé choisi dans le groupe formé par les composés comportant des radicaux alkyle en C₃ à C₈ et les composés comportant des groupes sulfate.

La phase stationnaire solide peut être sous forme de fibres ou de membranes ; elle est, de préférence, constituée par un matériau particulaire neutre, c'est-à-dire généralement considéré comme neutre en chromatographie, dont les particules ont une dimension moyenne inférieure à 2 mm, de préférence comprise entre 1 µm et 2 mm. Dans ce cas, la chromatographie est effectuée sur au moins une colonne.

La solution aqueuse brute d'albumine est, de préférence, une solution obtenue à partir de plasma sanguin en particulier humain par une méthode connue de type COHN, selon laquelle on fractionne ledit plasma par des traitements successifs par modification de la température et par action d'agent de précipitation pour obtenir, après séparation au moins partielle des facteurs VIII et IX et des γ - globulines, à des solutions d'albumine pour extraire l'agent de précipitation et recueillir la solution d'albumine brute.

La solution aqueuse brute d'albumine a, avantageusement, une concentration en albumine brute comprise entre 1 g et 300 g par litre et a un pH compris entre 6 et 8, de préférence entre 6,9 et 7,1.

Le composé comportant des radicaux alkyle en C₃ à C₈, qui charge la phase stationnaire, est, de préférence, un radical butyle. Le lit de remplissage de la (des) colonne(s) de chromatographie est alors notamment constitué par le produit commercialisé par la société "MERCK" sous la dénomination commerciale "FRACTOGEL TSK-BUTYL-650".

Le composé comportant des groupes sulfate qui charge la phase stationnaire est, de préférence, un dextran sulfate. Le lit de remplissage de la (des) colonne(s) de chromatographie est notamment constitué par le produit commercialisé sous la dénomination "DEXTRAN BEADS SULFATED" par la société "SIGMA".

Selon la présente invention, on fait, de préférence, subir à la solution aqueuse brute d'albumine au moins deux chromatographies, l'une au moins étant une chromatographie sur phase stationnaire chargée d'un composé comportant des radicaux alkyle en C₃ à C₈ et au moins une autre étant une chromatographie sur phase stationnaire chargée d'un composé comportant des groupes sulfate.

De façon avantageuse, on soumet la solution aqueuse brute d'albumine à une chromatographie sur phase stationnaire chargée en composé comportant des groupes sulfate, avant et après une chromatographie sur phase stationnaire chargée d'un composé comportant des radicaux alkyle en C₃ à C₈ ou inversement à une chromatographie sur phase stationnaire chargée en composé portant des radicaux alkyle en C₃ à C₈ avant et après une chromatographie sur phase stationnaire chargée en groupes sulfate.

On peut, avantageusement, utiliser directement les effluents d'une première chromatographie pour alimenter la chromatographie suivante. La température de chromatographie est de 1 à 30° C. Le débit de chromatographie sur colonnes est généralement compris entre 1 et 30 cm/heure.

Si nécessaire, on ajuste le pH de la solution aqueuse d'albumine obtenue comme effluent final de chromatographie à une valeur comprise entre 6,9 et 7,1 puis on la soumet à une filtration stérile, pour répondre aux besoins réglementaires de la Pharmacopée européenne.

Avant de soumettre une solution aqueuse d'albumine à une chromatographie sur phase stationnaire chargée en composé portant des radicaux alkyle en C₃ à C₈, on équilibre la (les) colonne(s) correspondante(s) avec une solution aqueuse saline ayant au plus 10 fois la force ionique de la solution aqueuse d'albumine. De la même façon, avant de soumettre une solution aqueuse d'albumine à une chromatographie sur phase stationnaire chargée en composé portant des groupes sulfate, on équilibre la (les) colonne(s) correspondante(s) avec une solution saline au plus isotonique par rapport à la solution d'albumine. De préférence, on utilise pour l'équilibrage de la (des) colonne(s) de chromatographie une solution de chlorure de sodium ou un tampon salin phosphate (PBS).

Dans le mode préféré de réalisation qui comporte deux ou trois chromatographies en série, le procédé selon l'invention ne nécessite aucun changement de tampon en cours de manipulation ni aucun changement de pH, les différentes chromatographies étant effectuées en série.

Dans le cas où la solution d'albumine traitée est une solution d'albumine plasmatique humaine préparée par le procédé Cohn ou un procédé dérivé, la solution d'albumine obtenue est stable, ne forme pratiquement pas de polymères à la chaleur et ne présente qu'une très faible coloration, ce qui correspond à la disparition des traces d'hémoglobine et d'hème. Lorsqu'on traite une solution d'albumine bovine par exemple à partir du produit "FLUKA n° 05480", l'albumine obtenue est beaucoup plus stable que l'albumine initiale. Par ailleurs, il est possible de traiter des solutions d'albumine injectable déclassées (par exemple pour trouble ou particules) pour leur redonner une bonne stabilité.

Pour mieux faire comprendre l'objet de l'invention, on va en décrire maintenant, à titre d'exemple purement illustratif et non limitatif, un mode de mise en oeuvre, les caractéristiques du produit obtenu étant représentées sur le dessin annexé.

Sur ce dessin :
- la figure 1 représente les profils de coloration après électrophorèse sur acétate de cellulose pour une albumine obtenue selon l'invention et une albumine de l'état de la technique (selon la méthode de Cohn) ;
- la figure 2 représente les courbes d'absorption de densité optique obtenues par filtration sur gel pour une albumine obtenue selon l'invention et une albumine de l'état de la technique (selon la méthode de Cohn);
- la figure 3 représente le spectre de densité optique obtenu pour une albumine obtenue selon l'invention et une albumine de l'état de la technique (selon la méthode de Cohn) ;
- la figure 4 représente la variation de la turbidité en fonction du temps de chauffage à 60° C pour une albumine obtenue selon l'invention et une albumine de l'état de la technique (selon la méthode de Cohn).

On utilise, comme matière première de départ, un plasma humain fractionné selon le procédé décrit par KISTLER et NITSCHMAN (Vox Sang. 7, 414-424 (1962)) ; ce procédé reprend l'essentiel de la méthode de Cohn précédemment décrite : les fractions de départ contenant l'albumine sont, soit le surnageant IV, soit le précipité V.

Lorsque l'on part du surnageant IV, qui comprend 40 % (en volume) d'éthanol et a un pH de 5,85 ± 0,05, on dilue de moitié le surnageant avec une solution de NaCl à 7 g/l. Le pH est ajusté à 7,45 ± 0,05 avec une solution de soude 1N. On pré-concentre l'albumine jusqu'à 90 g/l dans une cassette d'ultra filtration de type "Oméga" (Filtron) mise en oeuvre dans un système d'ultra-filtration "Minisette SS Cell NPT Cell" (Filtron Techn. Corp.). Cette cassette a une surface filtrante de 0,07 m² et un seuil de rétention de 30 KDa. La circulation est assurée par une pompe péristaltique à une pression qui varie de 2 x 10⁵ Pa au début de l'opération à 5 x 10⁵ Pa vers la fin. Lorsque l'on a atteint la concentration de 90 g/l en albumine, on ajoute à la solution d'albumine une solution de NaCl à 9 g/l et on continue à dialyser à volume constant jusqu'à obtenir un taux d'éthanol inférieur à 0,1 % en volume. Lorsque l'éthanol a été ainsi éliminé, on poursuit la dialyse pour concentrer la solution jusqu'à 200 g d'albumine par litre. On ajuste le pH à 7,10 ± 0,05 avec une solution d'acide chlorydrique 1N.

Lorsque l'on part du précipité V, on remet ledit précipité en suspension dans du sérum physiologique (solution de NaCl à 9 g par litre) en utilisant 4 litres de sérum physiologique par kilogramme de précipité. On filtre pour éliminer les grumeaux de précipité non remis en suspension au cours de l'agitation ; on ajuste le pH à 7,45 ± 0,05 avec une solution de soude 1N et on concentre la solution ainsi obtenue jusqu'à 90 g de protéines par litre en utilisant le même système de dialyse que précédemment décrit pour le surnageant IV. On poursuit alors la dialyse en rajoutant du sérum physiologique et en travaillant à volume constant jusqu'à obtenir un taux final d'éthanol inférieur à 0,1 % en volume. Lorsque l'éthanol a été ainsi éliminé, on poursuit la dialyse pour concentrer la solution jusqu'à 200 g de protéines par litre. On ajuste le pH à 7,1 ± 0,05 avec une solution d'acide chlorhydrique 1N.

La solution d'albumine ainsi préparée à partir du surnageant IV ou du précipité V est alors filtrée stérilement (filtre "Millex-GS" de 0,2 micron (Millipore)). On obtient ainsi la solution aqueuse brute d'albumine qui sera ensuite traitée par le procédé selon l'invention.

La solution aqueuse brute d'albumine subit d'abord une chromatographie sur phase stationnaire chargée en composé portant des groupes sulfate, dite chromatographie sur gel polysulfaté. Cette chromatographie est réalisée dans une colonne de 50 ml (2,5 cm x 10 cm) (Biorad), chargée avec un matériau particulaire vendu par la société "SIGMA" sous la dénomination commerciale "DEXTRAN BEADS SULFATED" La colonne est préalablement équilibrée en faisant passer dans le lit 3 volumes de colonne de sérum physiologique. La solution d'albumine est alors envoyée sur cette colonne et l'effluent est envoyé directement sur une deuxième colonne de chromatographie sur phase stationnaire chargée en radicaux en C₃ à C₈, dite chromatographie hydrophobe.

La chromatographie hydrophobe est réalisée dans une colonne identique à la précédente, dont le lit de chromatographie est constitué d'un matériau particulaire vendu par la société MERCK sous la dénomination commerciale "FRACTOGEL TSK BUTYL-650". Avant d'être utilisée, cette colonne est équilibrée en faisant passer dans le lit de chromatographie 3 volumes de colonne de sérum physiologique. Lorsqu'elle a été équilibrée, la colonne de chromatographie hydrophobe est branchée directement en série sur la sortie de la colonne de chromatographie sur gel polysulfaté. L'équilibrage des deux colonnes peut également être effectué en série.

Le déroulement de la chromatographie est suivi en mesurant la densité optique à 280 nm des fractions en sortie de colonne. L'effluent des deux colonnes de chromatographie en série est récupéré puis concentré par dialyse à 200 g d'albumine par litre ou dilué, dans du sérum physiologique, à 40 g d'albumine par litre suivant l'usage auquel on le destine ; le pH est ajusté à 7,00 ± 0,05 et l'effluent est filtré stérilement sur des filtres "Millex-GS" de 0,2 micron (Millipore). Le débit dans les deux colonnes montées en série est de 16 cm/heure ; les chromatographies sont effectuées à 20° C. En suivant le déroulement de la chromatographie, on constate que l'on peut charger jusqu'à 400 g d'albumine par litre de lit de chromatographie dans la première ou dans la deuxième colonne sans diminuer sensiblement l'efficacité de la séparation.

Le lit de la colonne de chromatographie sur gel polysulfaté est régénéré par un lavage avec deux volumes de colonne d'une solution de chlorure de sodium à 2 moles par litre, suivi par un lavage avec deux volumes de colonne d'une solution de chlorure de sodium à 1 mole par litre, à pH 9. La régénération du remplissage de la colonne de chromatographie hydrophobe s'effectue par lavage avec deux volumes de colonne d'une solution de soude 0,1 N.

On a constaté que la capacité de fixation des lits de chromatographie n'était pas affectée par 40 cycles successifs chromatographie/régénération.

On a évalué certaines caractéristiques de l'albumine obtenue par le procédé selon l'invention décrit ci-dessus et l'on fournit ci-après les résultats expérimentaux correspondants. On a comparé les propriétés de la solution aqueuse d'albumine avant passage sur les deux colonnes de chromatographie sur gel polysulfaté et hydrophobe et après passage sur ces deux colonnes. La solution avant passage est donc une albumine de l'état de la technique et la solution après passage est une albumine chromatographiée selon l'invention.

On prépare un échantillon à partir d'albumine de l'état de la technique et un échantillon à partir de l'albumine préparée selon l'invention. On ajoute à ces échantillons 10 mg de caprylate de sodium par gramme d'albumine et on chauffe l'échantillon à 60°C pendant 10 heures.

### 1 - Immunoélectrophorèse :

On a utilisé la méthode de GRABAR (Grabar P. et autres, Biochem. Biophys. Acta, 17, 67-75 (1955)). L'anti-sérum total a été préparé en immunisant un cheval avec du plasma humain complet. Pour l'albumine de l'état de la technique, on constate la présence d'une ligne de précipitation autour du réservoir de dépôt, ce qui correspond à la présence de "néo-antigènes" constitués de protéines dénaturées ; au contraire, cette ligne de précipitation n'existe pas pour l'albumine chromatographiée selon l'invention, ce qui démontre que le procédé selon l'invention permet de séparer de l'albumine des protéines contaminantes constituant des impuretés.

### 2 - Electrophorèse sur membrane d'acétate de cellulose :

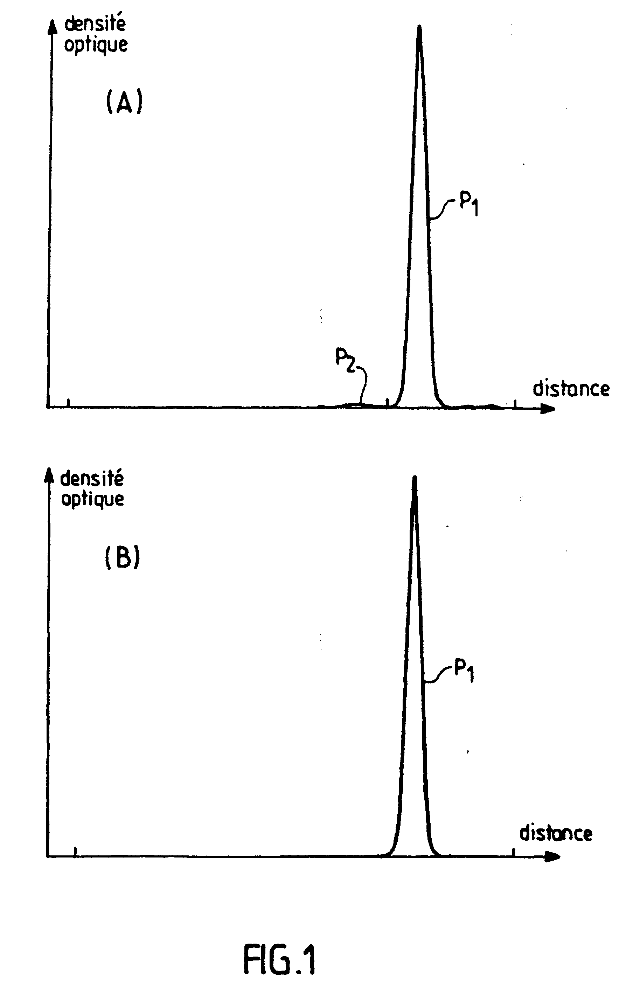

On a testé des solutions à 5 % de protéines comme décrit dans l'article ROELANDS J.F. et autres, Vox Sang., 26, 415-424 (1974). Les échantillons ont été testés sous 220 volts pendant 35 minutes avec un tampon "TRIS-VERONAL" à pH 9,2 à une force ionique de 0,05. Les membranes ont été révélées avec le colorant "PONCEAU S" et la quantification a été faite par densitométrie. Les courbes résultantes sont fournies sur la figure 1 : sur le graphique marqué A, on constate, à côté du pic principal P₁ qui correspond à l'albumine, un pic secondaire P₂ qui correspond à des impuretés, alors que sur le graphique B qui correspond à l'albumine selon l'invention, le pic P₁ se retrouve à l'identique mais le pic P₂ a disparu, ce qui montre l'efficacité du procédé de purification selon l'invention.

### 3 - Filtration sur gel :

On a utilisé une colonne "Biorad" (1,5 x 50 cm) avec un remplissage de "SEPHACRYL S 400 HR" vendu par la société "PHARMACIA LKB BIOTECHNOLOGY". Cette colonne est préalablement équilibrée avec un tampon phosphate à pH 7. On passe ensuite 20 mg d'albumine de chaque échantillon sur la colonne préparée ; le débit est de 1 ml par minute. On suit l'absorption optique à 280 nm sur les fractions chromatographiques (1,2 ml par fraction) au moyen d'un spectrophotomètre "Uvicon 810".

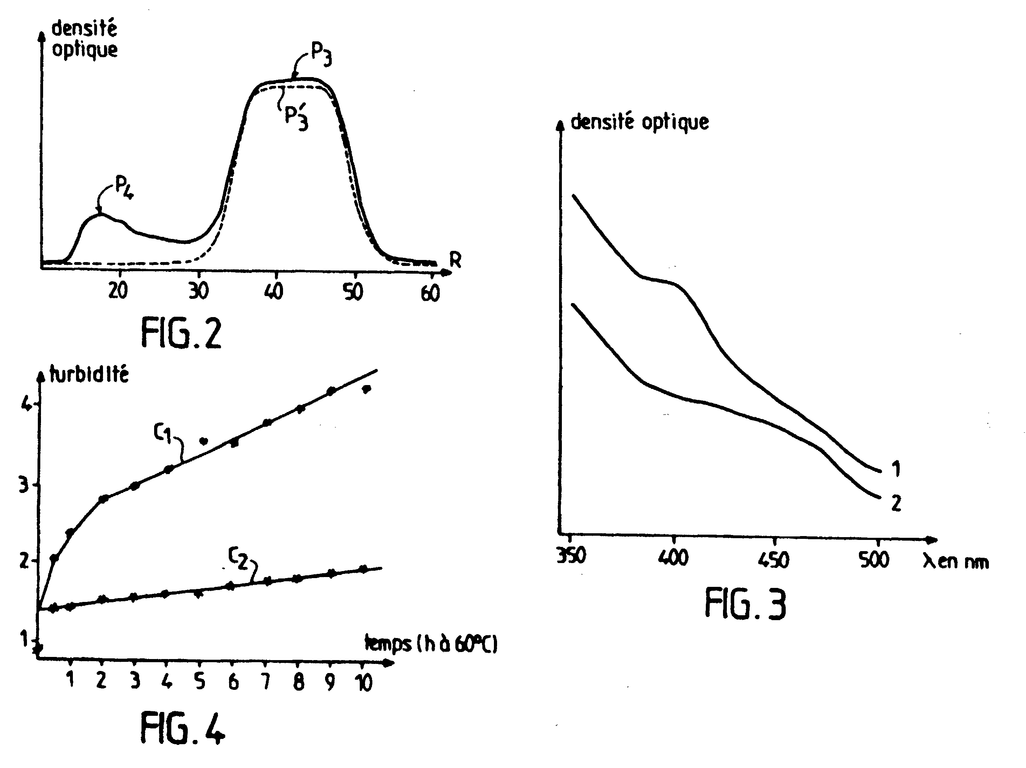

Les courbes expérimentales obtenues sont représentées sur la figure 2. La courbe en trait continu correspond à l'albumine de l'état de la technique et montre, à côté du pic P₃ correspondant à l'albumine, un pic P₄ correspondant à des impuretés contaminantes, dont des polymères. Au contraire, la courbe en pointillés correspond à l'albumine obtenue selon l'invention et, sur cette courbe, le pic P'₃, très voisin de P₃, correspond à l'albumine mais il n'y a pas de pic voisin ce qui démontre l'absence d'impureté contaminante.

### 4 - Spectre de densité optique :

On a étudié le spectre de densité optique entre 350 et 500 nm pour un échantillon d'albumine de l'état de la technique et un échantillon d'albumine obtenu selon l'invention (respectivement courbes 1 et 2 de la figure 3). On constate que, pour l'albumine obtenue selon l'invention, il y a absence d'absorption à 403 nm contrairement à ce qui se passe pour l'autre échantillon. On sait que l'hémoglobine absorbe à une longueur d'ondes lambda égale à 403 nm. On constate donc que l'albumine de l'état de la technique contient des traces d'hémoglobine, qui ne se retrouvent pas dans l'albumine obtenue selon l'invention ; cette dernière a effectivement une couleur moins intense, même après chauffage pendant 10 heures à 60° C avec un additif de stabilisation.

### 5 - Variation de turbidité :

On a étudié la variation de la turbidité en fonction du temps de chauffage à 60° C pour l'albumine de l'état de la technique et l'albumine obtenue selon l'invention. Les deux échantillons ont subi une addition de 1 % en poids de caprylate de sodium par rapport au poids d'albumine.

La figure 4 montre que l'échantillon d'albumine de l'état de la technique (courbe C₁) a une turbidité, qui évolue assez rapidement au cours du temps contrairement à l'échantillon d'albumine selon le procédé (courbe C₂). Cette variation de turbidité correspond à la formation de polymères. On constate donc que l'élimination des contaminants de l'albumine entraîne la réduction de la formation des polymères, qui apparaissent après le chauffage à 60° C pendant 10 heures et pendant le stockage prolongé des solutions d'albumine malgré l'addition de stabilisants, tels que le caprylate de sodium. Le procédé de préparation selon l'invention conduit à une solution d'albumine formant beaucoup moins de polymères, ce qui est particulièrement intéressant sur le plan commercial.

## Revendications

1. Procédé de purification d'une solution aqueuse d'albumine brute dans lequel on fixe des protéines contaminantes par chromatographie sur une phase stationnaire solide, la solution d'albumine purifiée étant recueillie comme effluent, **caractérisé par le fait qu'**on choisit comme phase stationnaire une phase neutre ou voisine de la neutralité chargée d'au moins un composé choisi dans le groupe formé par les composés comportant des radicaux alkyle en C₃ à C₈ et les composés comportant des groupes sulfate.

2. Procédé selon la revendication 1, **caractérisé par le fait que** la phase stationnaire est un matériau particulaire neutre dont les particules ont une dimension moyenne inférieure à 2 mm, de préférence comprise entre 1 µm et 2 mm et que la chromatographie est effectuée sur au moins une colonne.

3. Procédé selon l'une des revendications 1 ou 2, **caractérisé par le fait que** la solution aqueuse brute d'albumine est obtenue à partir de plasma sanguin humain par une méthode connue, selon laquelle on fractionne ledit plasma par des traitements successifs par modification de la température et par action d'un agent de précipitation pour aboutir, après séparation au moins partielle des facteurs VIII et IX et des γ-globulines, à des solutions d'albumine contenant l'agent de précipitation, que l'on dialyse pour extraire l'agent de précipitation et recueillir la solution d'albumine brute.

4. Procédé selon l'une des revendications 1 à 3, **caractérisé par le fait que** la solution d'albumine à purifier a une concentration en albumine brute comprise entre 1 g et 300 g par litre et a un pH compris entre 6 et 8, de préférence entre 6,9 et 7,1.

5. Procédé selon l'une des revendications 1 à 4, **caractérisé par le fait que** le composé comportant des radicaux alkyle en C₃ à C₈ est un composé comportant des radicaux butyle.

6. Procédé selon l'une des revendications 1 à 4, **caractérisé par le fait que** le composé comportant des groupes sulfate est un dextran sulfate.

7. Procédé selon l'une des revendications 1 à 6, **caractérisé par le fait qu'**on fait subir à la solution aqueuse d'albumine brute au moins deux chromatographies, l'une au moins étant une chromatographie sur phase stationnaire chargée d'un composé comportant des radicaux alkyle en C₃ à C₈ et au moins une autre étant une chromatographie sur phase stationnaire chargée d'un composé comportant des groupes sulfate.

8. Procédé selon la revendication 7, **caractérisé par le fait que** l'on soumet la solution d'albumine brute, d'abord à une (ou plusieurs) chromatographie(s) sur phase stationnaire chargée d'un composé comportant des groupes sulfate, puis à une (ou plusieurs) chromatographie(s) sur phase stationnaire chargée en radicaux alkyle en C₃ à C₈.

9. Procédé selon l'une des revendications 7 ou 8, **caractérisé par le fait qu'**on soumet la solution aqueuse brute d'albumine à une chromatographie sur phase stationnaire chargée en composés comportant des radicaux alkyle en C₃ à C₈, avant et après une chromatographie sur phase stationnaire chargée en composé comportant des groupes sulfate ou inversement.

10. Procédé selon l'une des revendications 7 à 9, **caractérisé par le fait qu'**on utilise directement l'effluent d'une première chromatographie pour alimenter la chromatographie suivante.

11. Procédé selon l'une des revendications 1 à 10, **caractérisé par le fait que** le débit de chromatographie est compris entre 1 et 30 cm/heure.

12. Procédé selon l'une des revendications 1 à 11, **caractérisé par le fait que** la température de chromatographie est comprise entre 1 et 30°C.

13. Procédé selon l'une des revendications 2 à 12, **caractérisé par le fait qu'**avant de soumettre la solution aqueuse d'albumine brute à une chromatographie sur phase stationnaire chargée en composés comportant des radicaux alkyle en C₃ à C₈, on équilibre la (les) colonne(s) correspondante(s) avec une solution aqueuse saline ayant au plus 10 fois la force ionique de la solution d'albumine à traiter.

14. Procédé selon l'une des revendications 2 à 12, **caractérisé par le fait qu'**avant de soumettre la solution aqueuse d'albumine brute à une chromatographie sur phase stationnaire chargée d'un composé comportant des groupes sulfate, on équilibre la (les) colonne(s) correspondante(s) avec une solution saline au plus isotonique par rapport à la solution d'albumine à traiter.

15. Procédé selon l'une des revendications 13 ou 14, **caractérisé par le fait qu'**on utilise, pour l'équilibrage de la (des) colonne(s), une solution de chlorure de sodium ou un tampon phosphate.

## Claims

1. Method for purifying an aqueous solution of raw albumin wherein contaminant proteins are bound by chromatography to a stationary solid phase, the purified albumin solution being collected as an effluent, **characterized in that** a neutral phase, or one in the region of neutrality, charged with at least one compound chosen from the group formed by compounds containing C₃ to C₈ alkyl radicals and compounds containing sulfate groups is chosen as the stationary phase.

2. Method according to Claim 1, **characterized in that** the stationary phase is a neutral particulate material the particles of which have an average size of less than 2 mm, preferably between 1 µm and 2 mm, and **in that** the chromatography is carried out on at least one column.

3. Method according to either of Claims 1 and 2, **characterized in that** the raw aqueous solution of albumin is obtained from human blood plasma by a known process, according to which the said plasma is fractionated by successive treatments by modifying the temperature and by the action of a precipitation agent in order to arrive, after at least partial separation of the factors VIII and IX and of the γ-globulins, at albumin solutions containing the precipitation agent, which are dialysed in order to extract precipitation agent and to collect the raw albumin solution.

4. Method according to one of Claims 1 to 3, **characterized in that** the albumin solution to be purified has a raw albumin concentration of between 1 g and 300 g per liter and has a pH of between 6 and 8, preferably between 6.9 and 7.1.

5. Method according to one of Claims 1 to 4, **characterized in that** the compound containing C₃ to C₈ alkyl radicals is a compound containing butyl radicals.

6. Method according to one of Claims 1 to 4, **characterized in that** the compound containing sulfate groups is a dextran sulfate.

7. Method according to one of Claims 1 to 6, **characterized in that** the aqueous solution of raw albumin is subjected to at least two chromatographies, at least one being a chroma tography on a stationary phase charged with a compound containing C₃ to C₈ alkyl radicals and at least one other being a chromatography on a stationary phase charged with a compound containing sulfate groups.

8. Method according to Claim 7, **characterized in that** the raw albumin solution is first subjected to one (or more) chromatography (or chromatographies) on a stationary phase charged with a compound containing sulfate groups, followed by one (or more) chromatography (or chromatographies) on a stationary phase charged with C₃ to C₈ alkyl radicals.

9. Method according to either of Claims 7 and 8, **characterized in that** the raw aqueous solution of albumin is subjected to a chromatography on a stationary phase charged with compounds containing C₃ to C₈ alkyl radicals, before and after a chromatography on a stationary phase charged with a compound containing sulfate groups, or vice versa.

10. Method according to one of Claims 7 to 9, **characterized in that** the effluent from a first chromatography is used directly to supply the following chromatography.

11. Method according to one of Claims 1 to 10, **characterized in that** the chromatography flow rate is between 1 and 30 cm/hour.

12. Method according to one of Claims 1 to 11, **characterized in that** the chromatography temperature is between 1 and 30°C.

13. Method according to one of Claims 2 to 12, **characterized in that**, before subjecting the aqueous solution of raw albumin to a chromatography on a stationary phase charged with compounds containing C₃ to C₈ alkyl radicals, the corresponding column(s) is (are) equilibrated with an aqueous saline solution having at most 10 times the ionic strength of the albumin solution to be treated.

14. Method according to one of Claims 2 to 12, **characterized in that**, before subjecting the aqueous solution of raw albumin to a chromatography on a stationary phase charged with a compound containing sulfate groups, the corresponding column(s) is (are) equilibrated with a saline solution which is at most isotonic relative to the albumin solution to be treated.

15. Method according to either of Claims 13 and 14, **characterized in that** a sodium chloride solution or a phosphate buffer is used to equilibrate the column(s).

## Patentansprüche

1. Verfahren zur Aufreinigung einer wässrigen Lösung rohen Albumins, wobei man kontaminierende Proteine mittels Chromatographie auf einer festen stationären Phase zurückhält und die Lösung aufgereinigten Albumins als Eluat gewinnt, **dadurch gekennzeichnet, dass** man als stationäre Phase eine neutrale oder nahezu neutrale Phase wählt, die mit wenigstens einer Verbindung beladen ist, welche unter C₃-C₈-Alkylreste aufweisenden Verbindungen und Sulfatgruppen aufweisenden Verbindungen ausgewählt ist.

2. Verfahren nach Anspruch 1, **dadurch gekennzeichnet, dass** die stationäre Phase ein neutrales partikuläres Material ist, dessen Partikel eine mittlere Abmessung unterhalb von 2 mm, vorzugsweise von 1 µm bis 2 mm, aufweisen, und dass die Chromatographie auf wenigstens einer Säule durchgeführt wird.

3. Verfahren nach einem der Ansprüche 1 oder 2, **dadurch gekennzeichnet, dass** die wässrige Lösung rohen Albumins ausgehend von menschlichem Blutplasma mit einem bekannten Verfahren erhalten wird, wonach man das Plasma sukzessive durch Änderung der Temperatur und durch Einwirkung eines Fällungsmittels behandelt, um im Anschluss an eine wenigstens partielle Abtrennung der Faktoren VIII und IX und von γ-Globulinen zu Fällungsmittel enthaltenden Albuminlösungen zu gelangen, die man dialysiert, um das Fällungsmittel zu extrahieren und die Lösung des rohen Albumins zu gewinnen.

4. Verfahren nach einem der Ansprüche 1 bis 3, **dadurch gekennzeichnet, dass** die aufzureinigende Albuminlösung eine Konzentration rohen Albumins von 1 g bis 300 g pro Liter und einen pH-Wert von 6 bis 8, vorzugsweise von 6,9 bis 7,1, aufweist.

5. Verfahren nach einem der Ansprüche 1 bis 4, **dadurch gekennzeichnet, dass** die C₃-C₈-Alkylreste aufweisende Verbindung eine Butylreste aufweisende Verbindung ist.

6. Verfahren nach einem der Ansprüche 1 bis 4, **dadurch gekennzeichnet, dass** die Sulfatgruppen aufweisende Verbindung ein Dextransulfat ist.

7. Verfahren nach einem der Ansprüche 1 bis 6, **dadurch gekennzeichnet, dass** man die wässrige Lösung rohen Albumins wenigstens zwei Chromatographien unterwirft, wobei es sich bei wenigstens einer um eine Chromatographie auf einer stationären Phase, die mit einer C₃-C₈-Alkylreste aufweisenden Verbindung beladen ist, und bei wenigstens einer weiteren um eine Chromatographie auf einer stationären Phase, die mit einer Sulfatgruppen aufweisenden Verbindung beladen ist, handelt.

8. Verfahren nach Anspruch 7, **dadurch gekennzeichnet, dass** man die Lösung rohen Albumins zunächst einer (oder mehreren) Chromatographie(n) auf einer stationären Phase, die mit einer Sulfatgruppen aufweisenden Verbindung beladen ist, und dann einer (oder mehreren) Chromatographie(n) auf einer stationären Phase, die mit C₃-C₈-Alkylresten beladen ist, unterwirft.

9. Verfahren nach einem der Ansprüche 7 oder 8, **dadurch gekennzeichnet, dass** man die wässrige Lösung rohen Albumins zunächst einer Chromatographie auf einer stationären Phase, die mit C₃-C₈-Alkylreste aufweisenden Verbindungen beladen ist, und anschließend einer Chromatographie auf einer stationären Phase, die mit Sulfatgruppen aufweisenden Verbindungen beladen ist, oder umgekehrt, unterwirft.

10. Verfahren nach einem der Ansprüche 7 bis 9, **dadurch gekennzeichnet, dass** man das Eluat einer ersten Chromatographie direkt verwendet, um die sich anschließende Chromatographie zu speisen.

11. Verfahren nach einem der Ansprüche 1 bis 10, **dadurch gekennzeichnet, dass** der Chromatographiefluss 1 bis 30 cm/Stunde beträgt.

12. Verfahren nach einem der Ansprüche 1 bis 11, **dadurch gekennzeichnet, dass** die Chromatographietemperatur 1 bis 30 °C beträgt.

13. Verfahren nach einem der Ansprüche 2 bis 12, **dadurch gekennzeichnet, dass** man die betreffende(n) Säule(n) mit einer wässrigen Salzlösung, welche höchstens die 10-fache lonenstärke der zu behandelnden Albuminlösung aufweist, äquilibriert, bevor man die wässrige Lösung des rohen Albumins einer Chromatographie auf einer stationären Phase, die mit C₃-C₈-Alkylresten aufweisenden Verbindungen beladen ist, unterwirft.

14. Verfahren nach einem der Ansprüche 2 bis 12, **dadurch gekennzeichnet, dass** man die betreffende(n) Säule(n) mit einer zu der zu behandelnden Albuminlösung höchstens isotonischen Salzlösung äquilibriert, bevor man die wässrige Lösung rohen Albumins einer Chromatographie auf einer stationären Phase, die mit einer Sulfatgruppen aufweisenden Verbindung beladen ist, unterwirft.

15. Verfahren nach einem der Ansprüche 13 oder 14, **dadurch gekennzeichnet, dass** man zur Äquilibrierung der Säule(n) eine Natriumchloridlösung oder einen Phosphatpuffer verwendet.
